# EUROPÄISCHE PATENTANMELDUNG

(11) **EP 1 380 312 A1**
(43) Veröffentlichungstag der Anmeldung: **14.01.2004**
(21) Anmeldenummer: 02028621.7
(22) Anmeldetag: 20.12.2002
(51) Int. Cl.: A61L 27/36, A61F 2/28

(54) **Verfahren zum Zubereiten eines Knochenimplantats**

(30) Priorität: 10.07.2002 EP 02015377
(71) Anmelder: Jakob, Karl, Dipl.-Ing.(FH), D-85598 Baldham (DE); Topor, Boris, Prof. Dr. habil. med., 2045 Chisinau (MD); Topor, Olga, Doz. Ph.D., 2045 Chisinau (MD)
(72) Erfinder: Topor, Boris, Prof., Dr., Habil., Med., 2045 Chisinau (MD)
(74) Vertreter: Körner, Ekkehard, Dipl.-Ing.

(57) **Zusammenfassung**

Zur Herstellung eines Knochenimplantats werden Knochen von Spendern in einer Reihe von Schritten mit Formaldehyd, Salzsäure, Natriumthiosulfat, Natriumchlorid, und weiteren Chemilkalien behandelt, um drin die angeborenen morphogenetischen Proteine zu bewahren.

## Beschreibung

Die Erfindung bezieht sich auf das Gebiet der morphoinduktiven Knochentransplantate, die in vielfältigen Bereichen am menschlichen Körper anwendbar sind, insbesondere für plastische Operationen in der Neurochirurgie, in der Otorhinolaryngologie, in der Kiefer- und Gesichtschirurgie, der Traumatologie, Orthopädie und Onkologie.

Knochendefekte am menschlichen Körper sind die Folge von Geburtsfehlern, von Unfällen oder Krankheiten. Solche Defekte können nicht nur aus Fehlstellungen oder Abnutzungen bestehen, sondern auch darin, dass Knochen oder Knochenteile fehlen.

Zur Behebung solcher Defekte gibt es Prothesen oder Ersatzstücke, von denen wohl die Metall- oder Kunststoffplatten für den Ersatz fehlender Teile der Schädeldecke und künstliche Gelenke dem breiten Publikum am meisten bekannt geworden sind. Es ist indessen auch bekannt, dass der Einsatz solcher körperfremden Ersatzstücke keineswegs eine ideale Lösung zur Behebung der entsprechenden Defekte darstellt. Unter medizinischen, insbesondere auch psychologischen Gesichtspunkten wäre einer Reparatur der Defekte durch körpereigene Kräfte, soweit dieses möglich ist, der Vorzug zu geben.

Die Erfindung geht von der Überlegung aus, morphogenetische Proteine, die die Natur für die Regelung des Prozesses der reparativen Osteogenese vorgesehen hat, für die Behandlung der vorgenannten Defekte nutzbar zu machen. Ihr liegt die Aufgabe zugrunde, ein Verfahren anzugeben, mit dem aus menschlichen Knochen, die einer Knochenbank entnommen sind, ein Implantat hergestellt werden kann, mit dem nach Applikation im menschlichen Körper der induktive Mechanismus der Regeneration und die Differierung der multipatenten Zellen in Knochenzellen in Gang gesetzt und das Implantat in das Knochengerüst durch körpereigene Heilungskräfte integriert werden kann.

Die Erfindung verwendet wesentliche Gedanken aus der früheren europäischen Patentanmeldung 02 015 377.1 der nämlichen Anmelder, die eine formlose Knochenpaste und ihr Herstellungsverfahren beschreibt, zielt hier aber auf ein Knochenimplantat und gibt ein Verfahren zu dessen Zubereitung an, das im Patentanspruch beschrieben ist.

Das erfindungsgemäße Verfahren wird nachfolgend im Detail erläutert.

Im erfindungsgemäßen Verfahren werden Röhrenknochen verwendet, die einer Knochenbank entnommen werden und von 25- bis 50-jährigen Spendern stammen. In diesem Alter sind die Knochen nämlich besonders reich an morphogenetischen Proteinen. Die Knochen werden mechanisch gereinigt, denn es wird nur das reine Knochenmaterial benötigt. Muskeln, Knochenhaut und Knochenmark müssen entfernt werden.

Die Knochen werden in einem inerten Behälter, der beispielsweise aus Glas besteht, mit einer 0,2 bis 0,5 %-igen, insbesondere 0,35 %-igen Formaldehydlösung mit einem Volumenverhältnis Formaldehydlösung zu Knochen von 1 : (5-10), vorzugsweise 1 : 5, übergossen, wobei die Formaldehydlösung aus 35 oder 25 %-igem, methanolfreiem, extrareinem Formaldehyd gewonnen ist, das in einer 0,85 bis 0,95 %-igen, vorzugsweise 0,9 %-igen Natriumchloridlösung gelöst wurde. Durch die methanolfreie Formaldehydlösung und die Natriumchloridlösung werden die Proteine im Knochen fixiert, ohne inaktiviert zu werden. Die Knochen bleiben 7 Tage bei einer Temperatur von 5 - 7 °C in dem Behälter, wobei die Formaldehydlösung täglich einmal gewechselt wird. Der Wechsel der Formaldehydlösung ist erforderlich, weil für die volle Fixierung der Proteine stets frische, freie Formaldehydmoleküle benötigt werden.

Nach Abschluß dieser Behandlung werden die Knochen mechanisch bearbeitet, um sie an Größe und Form der anatomischen und chirurgischen Besonderheiten des mit ihnen zu reparierenden Knochendefekts anzupassen. Es werden die Ränder abgeschnitten, ggf. Kerben eingebracht, Löcher und Kanäle gebohrt, wobei ggf. noch vorhandene Knochenmarkreste entfernt werden.

Anschließend werden die bearbeiteten Knochen 24 Stunden lang in einer 0,35%-igen Formaldehydlösung bei 5 - 7 °C gelagert und dann in einer 1 bis 3 %-igen, vorzugsweise 1,5 %-igen, chemisch reinen Salzsäure gelagert, wodurch ihre Phosphor- und Calciumsalze entfernt werden, die durch ihre Natur die Wirksamkeit der morphogenetischen Proteine blockieren. Die Behandlung in der Salzsäure erfolgt so lange, bis die Knochen einen vorbestimmten Grad der Demineralisierung erreicht haben, den man für die Bedingungen der jeweiligen chirurgischen Operation benötigt. Der Chirurg entscheidet über den Grad der Demineralisierung und bestellt das entsprechende Knochenmaterial; d.h. bei einem Armoder Beinknochen muss der Grad der Demineralisierung kleiner sein als beim Gesichtsoder Schädelknochen, wo eine Plastik notwendig ist.

Dann werden die bearbeiteten Knochen erneut 24 Stunden lang in einer 0,35%-igen Formaldehydlösung bei 5 - 7 °C gelagert und anschließend eine Stunde lang in einer 1 %-igen Natriumthiosulfat-Lösung behandelt, die alle 15 Minuten geschüttelt wird. Damit soll die Wirkung der im Knochen verbliebenen Salzsäuremoleküle neutralisiert werden.

Sodann werden die Knochen eine Stunde lang in einer 1,5 %-igen Ammoniakwasserlösung gelagert, die alle 15 Minuten geschüttelt wird. Mit ihr werden die Formaldehydmoleküle neutralisiert. Es folgt eine Behandlung der Knochen in einer 0,01 bis 0,1 %-igen Lösung von Chlorhexidinbigluconat, die als Grundlage einen 70%-igen Ethylalkohol hat, 48 Stunden lang bei einer Temperatur zwischen 5 und 7 °C, wobei die Lösung nach 1, 6 und 24 Stunden Einwirkungszeit gewechselt wird. Der Zweck dieser Maßnahme ist eine vollständige Sterilisation des Knochens. Schließlich werden die Knochen in eine isotonische 0,9 %-ige Lösung von Natriumchlorid gegeben, die nach 1, 2, 3, 4, 5, 6, 24 und 25 Stunden gewechselt und schließlich nach einer weiteren Stunde abgegossen wird. Mit dieser Maßnahme werden Fette und fettgebundene Proteine entfernt und die antigene Aktivität des zubereiteten Knochenimplantats reduziert. Schließlich wird die Natriumchloridlösung abgegossen.

Die zubereiteten Knochen werden bis zu ihrer Verwendung steril aufbewahrt, beispielsweise in Durchsichtigen Kunststoffbeuteln, und tiefgekühlt, vorzugsweise bei einer Temperatur von -20 °C aufbewahrt. Sie sind nach ihrer Zubereitung in dieser Form etwa 6 Monate lagerfähig.

Für den Transport, vorzugsweise in Thermotasche oder Thermosflasche, ist es günstig, wenn die Kühlkette nicht unterbrochen wird, doch ist eine Erwärmung auf bis zu 15 °C unschädlich, wenn sie nicht länger als drei Tage andauert.

Das erfindungsgemäße Verfahren ist relativ einfach auszuführen, in der Knochenmatrix bleiben alle angeboren Proteine erhalten, sowohl die morphogenetischen, die für den Beginn und die Regelung des Knochenregenerationsprozesses der reparativen Osteogenesis zuständig sind, als auch die nicht morphogenetischen, die die Regeneration unterstützen. Gleichzeitig sind alle antigenen Eigenschaften reduziert, was den Einbau des fremden Knochenmaterials in das Knochengerüst begünstigt, und sind toxische Reagenzien neutralisiert und beseitigt.

## Patentansprüche

1. Verfahren zum Zubereiten eines Knochenimplantats in folgenden Schritten:
a) Knochen von 25- bis 50-jährigen Spendern werden von Muskeln, Knochenhaut und Knochenmark befreit;
b) die Knochen werden in einem inerten Behälter mit einer 0,2 bis 0,5 %-igen Formaldehydlösung mit einem Volumenverhältnis Formaldehydlösung zu Knochen von 1 : (5-10) übergossen, wobei die Formaldehydlösung aus 35 oder 25 %-igem, methanolfreiem, extrareinem Formaldehyd gewonnen ist, das in einer 0,85 bis 0,95 %-igen Natriumchloridlösung gelöst wurde;
c) die Knochen bleiben 7 Tage bei einer Temperatur von 5 - 7 °C in dem Behälter, wobei die Formaldehydlösung täglich einmal gewechselt wird;
d) nach Abschluß der Behandlung im Schritt c) werden die Knochen mechanisch bearbeitet, um sie an Größe und Form der anatomischen und chirurgischen Besonderheiten des mit ihnen zu reparierenden Knochendefekts anzupassen, wobei ggf. noch vorhandene Knochenmarkreste entfernt werden;
e) anschließend werden die bearbeiteten Knochen 24 Stunden lang in einer 0,35%igen Formaldehydlösung bei 5 - 7 °C gelagert;
f) dann werden die Knochen in einer 1 bis 3 %-igen, chemisch reinen Salzsäure gelagert, bis sie einen vorbestimmten Grad der Demineralisierung erreicht haben, der für die Bedingungen der jeweiligen chirurgischen Operation erforderlich ist;
g) anschließend werden die bearbeiteten Knochen erneut 24 Stunden lang in einer 0,35%-igen Formaldehydlösung bei 5 - 7 °C gelagert;
h) dann werden die Knochen eine Stunde lang in einer 1 %-igen Natriumthiosulfat-Lösung behandelt, die alle 15 Minuten geschüttelt wird;
i) anschließend werden die Knochen eine Stunde lang in einer 1,5 %-igen Ammoniakwasserlösung gelagert, die alle 15 Minuten geschüttelt wird;
j) sodann werden die Knochen in einer 0,01 bis 0,1 %-igen Lösung von Chlorhexidinbigluconat, die als Grundlage einen 70%-igen Ethylalkohol hat, 48 Stunden lang bei einer Temperatur zwischen 5 und 7 °C gelagert, wobei die Lösung nach 1, 6 und 24 Stunden Einwirkungszeit gewechselt wird;
k) anschließend werden die Knochen in eine isotonische 0,9 %-ige Lösung von Natriumchlorid gegeben, die nach der 1., 2., 3., 4., 5., 6., 24. und 25. Stunde gewechselt und schließlich nach einer weiteren Stunde abgegossen wird.
